(19) Europäisches Patentamt
European Patent Office
Office européen des brevets

(11) Publication number: **0 191 514 B1**

# (12) EUROPEAN PATENT SPECIFICATION

(45) Date of publication of patent specification: **03.04.91**

(51) Int. Cl.5: **C07D 233/90**, C07D 403/06, A01N 43/50

(21) Application number: **86200092.4**

(22) Date of filing: **20.01.86**

The file contains technical information submitted after the application was filed and not included in this specification

## (54) Imidazole derivatives, their preparation and their use as fungicides.

(30) Priority: **31.01.85 GB 8502398**

(43) Date of publication of application:
**20.08.86 Bulletin 86/34**

(45) Publication of the grant of the patent:
**03.04.91 Bulletin 91/14**

(84) Designated Contracting States:
**AT BE CH DE FR GB IT LI LU NL SE**

(56) References cited:
**DE-A- 2 732 531**
**DE-A- 3 217 094**
**FR-A- 2 224 155**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Pettman, Roger Bruce**
**5 Glovers Crescent**
**Sittingbourne Kent(GB)**
Inventor: **Wells, Nicholas Secker**
**38 Agnew Road**
**Forest Hill London SE23(GB)**

(74) Representative: **Bennett, David Arthur Horder et al**
**4, York Road**
**London SE1 7NA(GB)**

**EP 0 191 514 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to imidazole derivatives, to a process for their preparation, and to the use of such derivatives as fungicides.

EP-A-91056 (BASF) discloses a class of 5-substituted-4-methylimidazole compounds of formula

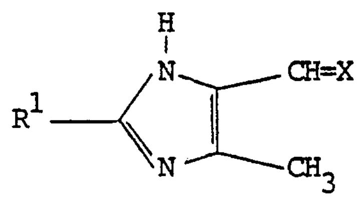

wherein X is <u>inter alia</u>

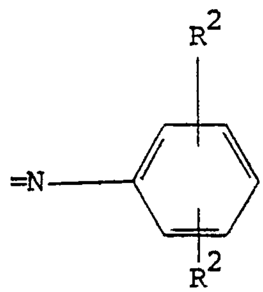

$R^1$ is hydrogen or an aliphatic moiety and each $R^2$ is independently hydrogen, an aliphatic moiety, a halogen atom or an alkoxy group. The compounds are disclosed as starting materials for plant protection compounds, dyestuffs and pharmaceuticals.

W. German Offenlegungsschrift 3 217 094 (Hoechst) discloses the preparation of various imidazole-5-carboxylic acid derivatives of formula

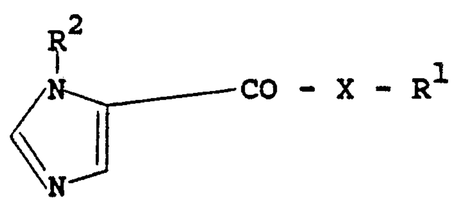

wherein $R^2$ is a phenyl or benzhydryl group optionally substituted by one or more substituents selected from halogen, $C_{1-4}$ alkoxy and $C_{1-4}$ alkyl moieties, X is 0, S or $-NR^1-$ and $R^1$ is H, phenyl, $C_{2-6}$ alkenyl, optionally substituted $C_{1-12}$ alkyl or, when X is 0 or S, a metal cation or ammonium. These derivatives are described as having fungicidal, herbicidal and plant-growth regulant activity, and the compounds wherein $R^2$ is an optionally substituted 2,6-dialkylphenyl group are claimed to be novel.

There has now been discovered a novel class of 5-substituted imidazole derivatives having useful fungicidal activity.

According to the present invention there is provided an imidazole-5-carboximidate of general formula

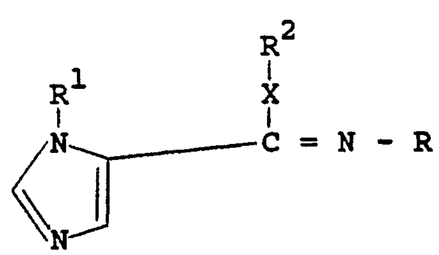

(I)

wherein R represents a phenyl group optionally substituted by a phenoxy group, a trifluoromethyl group, a methoxy group, a nitro group and/or by 1 to 5 halogen atoms; $R^1$ represents a $C_{1-6}$ alkyl group; $R^2$ represents a $C_{1-6}$ alkyl or haloalkyl group, a $C_{3-8}$ cycloalkyl group, a ($C_{3-8}$ cycloalkyl)methyl group, a $c_{3-6}$ alkenyl group, a phenyl group or a benzyl group; and X represents an oxygen or sulphur atom or a group $-NR^3-$, where $R^3$ represents a hydrogen atom or a methyl group or $R^2$ and $R^3$ together with the interjacent nitrogen atom represent a triazole or pyrrolidine ring.

Advantageously R is a phenyl group substituted by 1 to 3 halogen, preferably chlorine or fluorine, atoms or by a trifluoromethyl group and one or two halogen, preferably chlorine or fluorine atoms.

A particularly preferred sub-group of compounds of formula I is that wherein R is 2,4-dichlorophenyl, 2,4-difluorophenyl or 4-chloro-2-trifluoromethylphenyl, $R^1$ is a $C_{1-4}$ alkyl group, X is -O-, -S- or $-NCH_3-$ and $R^2$ is a $C_{1-4}$ alkyl, e.g. ethyl, isopropyl or isobutyl, or $C_{3-4}$ alkenyl, e.g. allyl or 3-but-1-enyl, group.

The present invention also provides a process for the preparation of an imidazole-5-carboximidate of formula I which comprises reacting a compound of formula

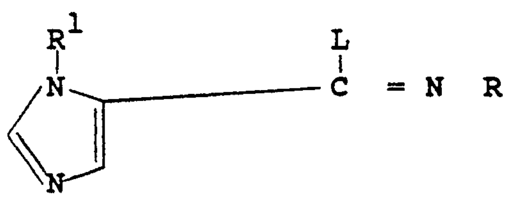

(II)

wherein R and $R^1$ are as defined above and L is a leaving group with a compound of formula

$$H-X-R^2 \qquad (III)$$

where $R^2$ and X are as defined above, in the presence of a base.

The leaving group L may conveniently be a chlorine or bromine atom.

In some cases, e.g. when X is -O-, the compound of formula III may advantageously be treated with the base prior to admixture with the compound of formula II. Thus for example when the compound of formula III is an alkanol the mixture of compound of formula III with base may be achieved by dissolving sodium metal in the alkanol or by reaction of the alkanol with sodium hydride. In cases where X is $-NR^3-$, the base may be an excess of compound of formula III or it may be a base such as pyridine. In cases where X is -S-, the base may conveniently be a base such as pyridine.

The above process may be effected in the absence of an additional, inert, solvent e.g. when the compound of formula III is in excess and the excess acts as a solvent, or in cases when e.g. pyridine is used as the base and itself acts as a solvent. Alternatively an additional, inert solvent may be present. Suitable solvents include dimethoxyethane, dimethylsulphoxide, N,M-dimethylformamide and tetrahydrofuran. Dimethoxyethane and dimethylsulphoxide have been found to be very suitable.

Compounds of formula II, wherein L is Cl or Br, may conveniently be prepared by reacting a compound of formula

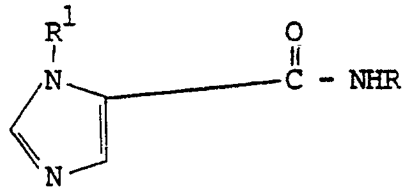

(IV)

where R and $R^1$ are as defined above, with a halogenating agent. Suitable halogenating agents include thionyl chloride, phosphorous pentachloride, phosphorous trichloride and phosphorous tribromide. Such reaction may if desired be effected in the presence of an inert solvent such as toluene, benzene, diethyl ether or tetrahydrofuran.

Compounds of formula IV are either known compounds or can be prepared by processes analogous to known processes, e.g. to processes described in R.G. Jones, J.Am.Chem.Soc. 71 (1949), 644, or in DE-A-3 217 094 referred to above.

Further in accordance with the invention there is provided a fungicidal composition which comprises a carrier and, as active ingredient, a carboximidate of formula I as defined above.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural silicas such as diatomaceous earths; magnesium silicates, for example talcs; magnesium aluminum silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or aralliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated from which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, at least one of which is a surface-active agent.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

A surface-active agent may be an emulsifying agent, a dispersing agent or a wetting agent; it nay be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p -octylphenol or p -octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecyl benzene sulponate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75%w of active ingredient and usually contain, in addition to solid inert carrier, 3-10%w of a dispersing agent and, where necessary, 0-10%w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing $\frac{1}{2}$-10%w of active ingredient. Granules are usually prepared to have a size between 10 and 100 BS mesh (1.676-0.152mm), and may be manufactured by agglomeration or impregnation techniques.

Generally, granules will contain $\frac{1}{2}$-25%w active ingredient and 0-10%w of additives such as stabilisers, slow release modifiers and binding agents. Emulsifiable concentrates usually contain, in addition to a solvent and, when necessary, co-solvent, 1-50%w/v active ingredient, 2-20%w/v emulsifiers and 0-20%w/v of other additives such as stabilisers, penetrants and corrosion inhibitors. Suspension concentrates are usually compounded so as to obtain a stable, non-sedimenting flowable product and usually contain 10-75%w of active ingredient, 0.5%-15%w of dispersing agents, 0.1-10%w of suspending agents such as

protective colloids and thixotropic agents, 0-10%w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

The compositions may also contain other ingredients, for example other compounds possessing pesticidal, especially insecticidal, acaricidal, herbicidal or fungicidal, properties.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The invention still further provides the use as a fungicide of a carboximidate of the general formula I as defined above, and a method for combating fungus at a locus, which comprises treating the locus, which nay for example be plants subject to or subjected to fungal attack, seeds of such plants or the medium in which such plants are growing or are to be grown, with such a derivative.

The present invention is of wide applicability in the protection of crop plants against fungal attack. Typical crops which may be protected include vines, grain crops such as wheat and barley, rice, beans and apples. The duration of protection is normally dependent on the individual compound selected, and also a variety of external factors, such as climate, whose impact is normally mitigated by the use of a suitable formulation. Application rates may typically be in the range 0.1 to 10 kg active ingredient per hectare (kg/ha), preferably 0.1 to 1 kg/ha.

The invention will be further understood from the following Examples.

EXAMPLE 1

Preparation of 1-methylpropyl N-(2,4-dichlorophenyl)-1-isopropylimidazole-5-carboximidate

(a) N-(2,4-dichlorophenyl)-1-isopropyl imidazole-5 carboxamide

1-Isopropyl-5-imidazolecarboxylic acid (2.1g) was heated under reflux in thionyl chloride (35ml) for 2½ hours. Excess thionyl chloride was evaporated off and the residue was added to a solution of 2,4-dichloroaniline (2.2g) in dry pyridine (25ml). The reaction was refluxed for 18 hours, allowed to cool and poured onto ice. The aqueous solution was extracted with ethyl acetate (3x100ml) and the combined organic extracts were washed with water (3x200ml) and dried ($MgSO_4$) and the solvent was evaporated off under reduced pressure leaving a brown oil. Column chromatography on silica using chloroform as eluant afforded a colourless oil which crystallised on scratching in ether/petroleum ether to afford white crystals of N-(2,4-dichlorophenyl)-1-isopropyl imidazole-5-carboxamide (2.4g, 59%), m.p. 77-78° C;

$C_{13}H_{13}Cl_2N_3O$ requires: C, 52.3; H, 4.4; N, 14.1%
Found : C, 52.3; H, 4.3; N, 13.9%

(B) 1-Methylpropyl N-(2,4-dichlorophenyl)-1-isopropyl imidazole-5-carboximidate N-(2,4-Dichlorophenyl)-1-isopropyl imidazole-5-carboxamide (1.7g) was refluxed in thionyl chloride (40ml) for 3 hours. Excess thionyl chloride was evaporated off under reduced pressure and the solid residue was suspended in dry dimethoxyethane (125ml). Sodium (0.3g) was dissolved in butan-2-ol (30ml) and this solution was added to the dimethoxyethane suspension. The reaction mixture was stirred and refluxed for 1 hour. After cooling to ambient temperature, the solvent was evaporated off under reduced pressure and the residue was taken up in chloroform and washed with water. The organic layer was dried ($MgSO_4$) and the solvent was evaporated off under reduced pressure to leave a brown oil. Column chromatography on silica using choroform as eluant afforded a yellow oil which crystallised on standing to give 1-methylpropyl N-(2,4-dichlorophenyl)-1-isopropyl imidazole-5-carboximidate (1.2g, 59%) m.p. 86-87° C;

$C_{17}H_{21}Cl_2N_3O$ requires: C, 57.6; H, 5.9; N, 11.9
Found : C, 57.6; H, 6.1; N, 11.7

EXAMPLE 2

Preparation of 1-methylpropyl-N(2,4,6-trichlorophenyl)-1-methylimidazole-5-carboximidate

(a) 1-Chloro-N-(2,4,6-trichlorophenyl)-1-methylimidazole-5-carboximidate

N-(2,4,6-Trichlorophenyl)-1-methylimidazole-5-carboxamide (1g) (prepared by a process similar to that of Example 1a) was refluxed in thionyl chloride (40ml) for 3 hours. Excess thionyl chloride was evaporated off and the residue was diluted with sodium bicarbonate solution and extracted with ether. The combined ether extracts were washed with water and dried ($MgSO_4$). The solvent was evaporated off to leave an oil which slowly solidified to a white solid of 1-chloro-N-(2,4,6-trichlorophenyl)-1-methyl imidazole-5-carboximidate (0.29g, 24%), m.p. 93-95 °C;

$C_{11}H_7Cl_4N_3$ requires: C, 40.9; H, 2.2; N, 13.0
Found : C, 41.2.; H, 2.2; N, 12.6

(b) 1-Methylpropyl-(2,4,6-trichlorophenyl)-1-methylimidazole-5-carboximidate

1-Chloro-N-(2,4,6-trichorophenyl)-1-methyl imidazole-5-carboximidate (1.0g) was suspended in dry dimethoxyethane (25ml). A solution of sodium (0.3g) in butan-2-ol (30ml) was added thereto and the reaction mixture was stirred and refluxed for 18 hours. On cooling, the solvent was evaporated off under reduced pressure and the residue taken up in chloroform and washed with water. The organic layer was dried ($MgSO_4$) and solvent was evaporated off under reduced pressure to leave an oil. Flash chromatography on silica using ether as eluant afforded 1-methylpropyl-N(2,4,6-trichlorophenyl)-1-methyl imidazole-5-carboximidate (0.49g, 45%), m.p. 90-92 °C.

EXAMPLE 3

1-1(1,2,4-triazolyl)-N-(2,4-dichlorophenyl)-1-methyl imidazole-5-carboximidate

N-(2,4-Dichlorophenyl)-1-methylimidazole-5-carboxamide (1.0g) was refluxed in thionyl chloride (25ml) for 2 hours. The excess thionyl chloride was evaporated off under reduced pressure and the solid residue was treated with 1,2,4-triazole (0.3g) in dry pyridine (15ml). The reaction mixture was refluxed for 2 hours and the pyridine was then evaporated off under reduced pressure. The resulting residue was taken up in chloroform, washed with water and dried ($MgSO_4$). The solvent was evaporated off to yield a yellow solid. Column chromatography on silica using 10%v/v methanol-ethyl acetate afforded 1-1(1,2,4-triazolyl)-N-(2,4-dichlorophenyl)-1-methyl imidazole-5-carboximidate as a yellow solid (0.3g, 25%), m.p. 129-131 °C;

$C_{13}H_{10}Cl_2N_6$ requires: C, 48.6; N, 3.1; N, 26.2
Found : C, 48.0; H, 3.2; N, 25.9

EXAMPLE 4

1-Methylpropyl-N-(2,4-dichlorophenyl)-1-methyl imidazole-5-carboxamidate

N-(2,4-Dichlorophenyl)-1-methylimidazole-5-carboximidate (1.0g) was refluxed in thionyl chloride (15ml) for 2 hours. The excess thionyl chloride was evaporated off, the residue was treated with sec-butylamine (20ml) and the resulting mixture was stirred under reflux for 1½ hours. Excess sec-butylamine was evaporated off under reduced pressure. The resulting residue was taken up in chloroform, washed with water, dried ($MgSO_4$) and the solvent was evaporated off under reduced pressure. The residual oil was chromatographed on silica using 4% v/v methanol-chloroform to afford 1-methylpropyl-N-(2,4-dichlorophenyl)-1-methyl imidazole-5-carboxamidate (1.0g, 83%) as a pale yellow oil.

Analysis: $C_{15}H_{18}Cl_2N_4$ requires: H, 5.5; N, 17.2
Found : H, 5.0; N, 15.4

EXAMPLE 5

1-Methylpropyl-N-(2,4-dichlorophenyl)-1-methyl imidazole-5-carboxthioimidate

N-(2,4-Dichlorophenyl)-1-methylimidazole-5-carboxamide (1.0g) was refluxed in thionyl chloride (25ml) for 2 hours. The excess thionyl chloride was evaporated off under reduced pressure, the solid residue was treated with sec-butyl mercaptan (1.2g) in dry pyridine (20ml) and the resulting mixture was refluxed for 18 hours. On cooling, the solvent was evaporated off, the resulting residue was taken up in ether, washed with water and dried ($MgSO_4$), and the solvent was evaporated off under reduced pressure. The resulting semi-solid residue was chromatographed on silica using 2% v/v methanol-chloroform as eluant to afford 1-methylpropyl-N-(2,4-dichlorophenyl)-1-methyl imidazole-5-carboxthioimidate (0.9g, 71%) as yellow oil.

Analysis: $C_{15}H_{17}Cl_2N_3S$ requires: H, 5.0; N, 12.3
Found : H, 5.2; N, 11.3

EXAMPLE 6

2,2-Dimethylpropyl N-(2,4-dichlorophenyl)-1-methylimidazole-5-carboximidate

N-(2,4-Dichlorophenyl)-1-methylimidazole-5-carboxamide (1.0g) was refluxed in thionyl chloride (25ml) for 2 hours. The excess thionyl chloride was evaporated off under reduced pressure to leave 1-chloro-N-(2,4-dichlorophenyl)-1-methylimidazole-5-carboximidate as a solid residue.

To a stirred solution of dry dimethylsulphoxide (50ml) containing sodium hydride (0.21g, 50% dispersion in oil, washed with hexane) under nitrogen was added neopentan-1-ol (0.65g) and the reaction mixture was stirred at 40°C for 1 hour. Sodium iodide (0.05g) was added to the reaction mixture and the 1-chloro-N-(2,4-dichlorophenyl)-1-methylimidazole-5-carboximidate dissolved in dry dimethylsulphoxide (50ml) was then added dropwise thereto. The reaction mixture was stirred at 40°C for 18 hours. On cooling, the reaction was poured into water and extracted with other. The combined ether extracts were washed with water, dried ($MgSO_4$) and the solvent evaporated off to leave a pale yellow oil. Column chromatography on silica using ether as eluant afforded 2,2-dimethylpropyl N-(2,4-dichlorophenyl)-1-methylimidazole-5-carboximidate (0.35g, 28%). m.p. 108-110°C.

$C_{16}H_{19}Cl_2N_3O$ requires: C, 56.5; H, 5.6; N, 12.4
Found : C, 55.7; H, 5.8; N, 12.1

EXAMPLES 7 to 27

By processes similar to those of Examples 1 to 6 were prepared a number of other compounds of the invention, as detailed in Table I following.

TABLE I

| Example | $R^1$ | X | $R^2$ | R | m.p. (°C) | Analysis Found (Theory) |
|---|---|---|---|---|---|---|
| 7 | $CH_3-$ | O | $(C_2H_5)(CH_3)CH-$ | 2,4-dichlorophenyl | oil | 5.0 H, 12.5 N<br>(5.2 H, 12.9 N) |
| 8 | $CH_3CH_2CH_2-$ | O | $(C_2H_5)(CH_3)CH-$ | 2,4-dichlorophenyl | oil | 56.6 C, 5.9 H, 11.7 N<br>(57.6 C, 5.9 H, 11.9 N) |
| 9 | phenyl | O | $(C_2H_5)(CH_3)CH-$ | 2,4-dichlorophenyl | 85-88 | 61.4 C, 4.9 H, 10.6 N<br>(61.9 C, 4.9 H, 10.8 N) |
| 10 | $CH_3-$ | O | $CH_3-$ | 2,4-dichlorophenyl | 123-4 | 50.5 C, 3.9 H, 14.7 N<br>(50.7 C, 3.9 H, 14.8 N) |
| 11 | benzyl | O | $(C_2H_5)(CH_3)CH-$ | 2,4-dichlorophenyl | oil | 60.5 C, 5.1 H, 9.9 N<br>(62.7 C, 5.2 H, 10.4 N) |
| 12 | $CH_3-$ | O | $(C_2H_5)(CH_3)CH-$ | 4-chlorophenyl | oil | 61.5 C, 6.5 H, 14.1 N<br>(61.8 C, 6.2 H, 14.4 N) |
| 13 | $CH_3-$ | O | $CH_3CH_2CH_2CH_2-$ | 4-chlorophenyl | 70-72 | 61.4 C, 6.4 H, 14.2 N<br>(61.8 C, 6.2 H, 14.4 N) |
| 14 | $CH_3-$ | O | $CH_3CH_2CH_2-$ | 2,4-dichlorophenyl | oil | 53.4 C, 4.6 H, 13.0 N<br>(53.8 C, 4.8 H, 13.5 N) |
| 15 | $CH_3-$ | O | $(CH_3)_2CH-$ | 2,4-dichlorophenyl | oil | 52.8 C, 5.1 H, 13.2 N<br>(53.8 C, 4.8 H, 13.5 N) |

TABLE I continued

| Example | $R^1$ | X | $R^2$ | R | m.p. (°C) | Analysis Found (Theory) |
|---|---|---|---|---|---|---|
| 16 | $CH_3$- | O | $(CH_3)_2CH.CH_2$- | 2,4-dichlorophenyl | oil | 54.4 C, 5.2 H, 12.1 N<br>(55.2 C, 5.2 H, 12.9 N) |
| 17 | $CH_3$- | -N-pyrrolyl | | 2,4-dichlorophenyl | oil | 54.8 C, 4.8 H, 17.0 N<br>(55.7 C, 5.0 H, 17.3 N) |
| 18 | $CH_3$- | $CH_3$-$NCH_3$- | | 2,4-dichlorophenyl | oil | 50.3 C, 4.4 H, 17.6 N<br>(52.5 C, 4.7 H, 18.8 N) |
| 19 | $CH_3$- | O | $(CH_3)_2CH$- | 4-phenoxyphenyl | oil | 5.7 H, 11.3 N<br>(6.3 H, 12.5 N) |
| 20 | $CH_3$- | S | $(CH_3)_3C$- | 2,4-dichlorophenyl | 110-111° | 52.8 C, 5.0 H, 11.8 N<br>(52.6 C, 5.0 H, 12.3 N) |
| 21 | $CH_3$- | O | $(CH_3)_2CH$- | 4-chloro-2-trifluoromethylphenyl | oil | 51.7 C, 4.1 H, 12.1 N<br>(52.1 C, 4.3 H, 12.15 N) |
| 22 | $CH_3$- | O | $(C_2H_3)(CH_3)CH$- | 4-chloro-2-trifluoromethylphenyl | oil | 53.1 C, 4.6 H, 11.6 N<br>(53.4 C, 4.7 H, 11.7 N) |
| 23 | $CH_3$- | S | $(CH_3)_2CH$- | 2,4-dichlorophenyl | oil | 50.9 C, 4.3 H, 11.9 N<br>(51.2 C, 4.6 H, 12.8 N) |
| 24 | $CH_3$- | O | $CH_3CH_2$- | 2,4-dichlorophenyl | oil | 52.4 C, 4.5 H, 13.6 N<br>(52.7 C, 4.4 H, 14.2 N) |

TABLE I continued

| Example | $R^1$ | X | $R^2$ | R | m.p. (°C) | Analysis Found (Theory) |
|---|---|---|---|---|---|---|
| 25 | $CH_3$- | O | benzyl | 2,4-dichlorophenyl | 94-96 | 61.7 C, 4.5 H, 11.8 N<br>(60.0 C, 4.2 H, 11.7 N) |
| 26 | $CH_3$- | O | phenyl | 2,4-dichlorophenyl | oil | 58.9 C, 3.8 H, 12.1 N<br>(55.8 C, 3.7 H, 11.0 N) |
| 27 | $CH_3$- | O | $CH_3(CH_2)_4$- | 2,4-dichlorophenyl | oil | 56.1 C, 5.6 H, 12.5 N<br>(56.5 C, 5.6 H, 12.4 N) |
| 28 | $(CH_3)_2CH.CH_2$- | O | $(C_2H_5)(CH_3)CH$- | 2,4-dichlorophenyl | oil | 58.1 C, 6.1 H, 11.4 N<br>(58.7 C, 6.3 H, 11.4 N) |
| 29 | $CH_3$- | O | $CH_2$=CH.$CH_2$- | 2,4-dichlorophenyl | oil | 53.6 C, 4.2 H, 13.2 N<br>(54.2 C, 4.2 H, 13.6 N) |
| 30 | $CH_3$- | O | $CF_3CH_2$- | 2,4-dichlorophenyl | oil | 44.3 C, 2.9 H, 11.8 N<br>(44.3 C, 2.8 H, 11.9 N) |
| 31 | $CH_3$- | O | $(CH_2=CH)(CH_3)CH$- | 2,4-dichlorophenyl | oil | 54.5 C, 4.7 H, 12.6 N<br>(55.5 C, 4.6 H, 13.0 N) |
| 32 | $CH_3$- | O | $CH_2FCH_2$- | 2,4-dichlorophenyl | oil | 46.8 C, 3.9 H, 12.7 N<br>(49.4 C, 3.8 H, 13.3 N) |
| 33 | $CH_3$- | O | cyclopropylmethyl | 2,4-dichlorophenyl | oil | 54.2 C, 4.6 H, 12.4 N<br>(55.5 C, 4.6 H, 13.0 N) |
| 34 | $(CH_3)_2CH$- | S | $(CH_3)_3C$- | 2,4-dichlorophenyl | oil | 50.2 C, 5.2 H, 10.1 N<br>(55.1 C, 5.7 H, 11.4 N) |

TABLE I continued

| Example | $R^1$ | X | $R^2$ | R | m.p. (°C) | Analysis Found (Theory) |
|---|---|---|---|---|---|---|
| 35 | $(CH_3)_2CH-$ | O | $(CH_3)_2CH-$ | 2,4-dichlorophenyl | oil | 56.2 C, 5.6 H, 12.1 N<br>(56.5 C, 5.6 H, 12.4 N) |
| 36 | $(CH_3)_2CH-$ | O | $CH_3CH_2CH_2-$ | 2,4-dichlorophenyl | oil | 55.9 C, 5.5 H, 12.0 N<br>(56.5 C, 5.6 H, 12.4 N) |
| 37 | $(CH_3)_2CH-$ | O | $(CH_3)_3CCH_2-$ | 2,4-dichlorophenyl | 87-89 | 58.7 C, 6.2 H, 11.4 N<br>(58.7 C, 6.3 H, 11.4 N) |
| 38 | $CH_3-$ | O | cyclohexyl | 2,4-dichlorophenyl | oil | 55.2 C, 5.6 H, 10.0 N<br>(58.0 C, 5.4 H, 11.9 N) |
| 39 | $(CH_3)_2CH-$ | O | benzyl | 2,4-dichlorophenyl | oil | 61.1 C, 5.1 H, 10.2 N<br>(61.9 C, 4.9 H, 10.8 N) |
| 40 | $CH_3-$ | O | $(CH_3)_2CH-$ | 2,4-difluorophenyl | oil | 60.1 C, 5.9 H, 15.0 N<br>(60.2 C, 5.4 H, 15.0 N) |
| 41 | $CH_3-$ | O | $CH_3CH_2CH_2-$ | 2,4-difluorophenyl | oil | 60.4 C, 5.8 H, 14.5 N<br>(60.2 C, 5.4 H, 15.0 N) |
| 42 | $CH_3-$ | O | $(C_2H_5)(CH_3)CH-$ | 2,4-difluorophenyl | oil | 60.4 C, 6.0 H, 13.4 N<br>(61.4 C, 5.8 H, 14.3 N) |

TABLE I continued

| Example | $R^1$ | X | $R^2$ | R | m.p. (°C) | Analysis Found (Theory) |
|---|---|---|---|---|---|---|
| 43 | $CH_3$- | O | $(C_2H_5)(CH_3)CH$- | 3,4-dichlorophenyl | oil | 52.2 C, 5.0 H, 12.0 N<br>(55.2 C, 5.2 H, 12.9 N) |
| 44 | $CH_3$- | O | $(C_2H_5)(CH_3)CH$- | 4-chloro-2-nitrophenyl | oil | 53.4 C, 5.1 H, 16.5 N<br>(53.6 C, 5.1 H, 16.7 N) |
| 45 | $CH_3$- | O | $(C_2H_5)(CH_3)CH$- | 4-chloro-2-methoxyphenyl | oil | 58.8 C, 6.5 H, 12.6 N<br>(59.8 C, 6.2 H, 13.1 N) |

EXAMPLE 46

The fungicidal activity of compounds of the invention was investigated by means of the following tests.

(a) Direct protectant activity against vine downy mildew (Plasmopara viticola; Pvp)

The test is a direct protectant one, using a foliar spray. The lower surfaces of leaves of whole vine plants (cv Cabernet Sauvignon) are sprayed with a solution of active material in 1:1v/v water/acetone containing 0.04%w "Triton x-155" (trade mark) (octylphenol polyoxyethylene surfactant), at a dosage of 1 kilogram of active material per hectare using a track sprayer which delivers 620 1/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(b) Direct protectant activity against vine grey mould (Botrytis cinerea; Bcp)

The test is a direct protectant one using a foliar spray and is effected as described under (a), with the difference that the leaves are inoculated by spraying with an aqueous solution containing $10^5$ conidia/ml.

(c) Activity against wheat leafspot (Leptosphaeria nodorum; ` ia nodo; Ln.)

The test is a direct antisporulant one, using a foliar spray. Leaves of wheat plants (cv Mardler), at the single leaf stage, are inoculated by spraying with an aqueous suspension containing 8 x $10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(d) Activity against barley powdery mildew (Erysiphe graminis f.sp. hordei; Eg)

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1kg. of active material per hectare using a track sprayer as described under (a). After drying, plants are returned to a compartment at ambient temperature and humidity for up to 7 days, followed by assessment. Assessment is based on the percentage of leaf area covered by sporulation compared with that on leaves of control plants.

(e) Activity against apple powdery mildew (Podosphaera leucotricha; Pl)

The test is a direct anti-sporulant one using a foliar spray. The upper surfaces of leaves of whole apply seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer as described under (a). After drying the plants are returned to a compartment at ambient temperature and humidity for up to 9 days, followed by assessment. Assessment is based on the percentage of the leaf area covered by sporulation compared with that on

leaves of control plants.

(f) Activity against broad bean rust (Uromyces fabae Uf)

The test is a direct antisporulant one using foliar spray. Pots containing 1 plant per pot were inoculated by spraying an aqueous suspension, containing $5x10^4$ spores/ml plus a little "Triton X-155", onto the upper surface of each leaf 20-24 hours before treatment with test compound. The inoculated plants were kept overnight in a high humidity compartment, dried at glass-house ambient temperature and then sprayed, on the leaf upper surface, at a dosage of 1kg/ha of active material using a track sprayer as described under (a) . After treatment the plants were kept at glass-house temperature and assessment made 11-14 days after treatment. Symptoms are assessed on the relative density of sporulating pustules per plant compared with that on control plants.

(g) Activity against rice leaf blast (Pyricularia oryzae Po)

The test is a direct eradicant one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying at a dosage of 1kg of active material per hectare using a track sprayer as described under (a). After treatment the plants are kept in a rice compartment at 25-30°C and high humidity. Assessments are made 4-5 days after treatment and are based on the density of necrotic lesions and the degree of withering when compared with control plants. The exent of disease control inall the above tests is expressed as a rating compared with a diluent sprayed control according to the criteria:-

0 = less than 50% disease control
1 = about 50-80% disease control
2 = greater than 80% disease control

Results of the above tests are given in Table II following:

TABLE II

| Compound Example | Fungicidal Activity | | | | | | |
|---|---|---|---|---|---|---|---|
| | Pvp | Bcp | Ln | Eg | Pl | Uf | Po |
| 1 | 2 | | | 2 | | 1 | |
| 2 | 2 | | | 2 | | | 1 |
| 3 | | | | 2 | | | 1 |
| 4 | 1 | 1 | | 2 | | | |
| 5 | 2 | | | 2 | | 1 | |
| 6 | | | | 2 | | 2 | 2 |
| 7 | | | | 2 | | 2 | |
| 8 | | 1 | | 2 | | 2 | |
| 9 | 1 | | | 2 | 2 | | |
| 10 | | | 2 | 2 | | | |
| 11 | 2 | | | 2 | 2 | | |
| 12 | | | | 2 | | 2 | |
| 13 | | | 1 | 2 | | 1 | 2 |
| 14 | | | | 2 | | 2 | |
| 15 | 2 | | 2 | 2 | | 2 | 2 |
| 16 | 2 | | 1 | 2 | | 2 | 2 |
| 17 | | | | 2 | | | |
| 18 | 2 | 2 | | 2 | | | |
| 19 | 1 | | | 2 | | | |
| 20 | 2 | 2 | 2 | 2 | | 2 | |
| 21 | 2 | | 2 | 2 | | | 2 |
| 22 | 2 | | 1 | 2 | | | 2 |
| 23 | 2 | | 1 | 2 | | 2 | |
| 24 | 1 | | 2 | 2 | | 2 | 2 |
| 25 | 2 | | 1 | 2 | | 2 | |
| 26 | | 2 | | 2 | | | |

TABLE II (continued)

| Compound Example | Fungicidal Activity | | | | | | |
|---|---|---|---|---|---|---|---|
| | Pvp | Bcp | Ln | Eg | Pl | Uf | Po |
| 27 | | | | 2 | | 2 | |
| 28 | | | | 2 | 2 | | 2 |
| 29 | 2 | 2 | 2 | 2 | 2 | | 2 |
| 30 | | 2 | | 2 | | 2 | |
| 31 | | | 2 | 2 | 2 | 1 | 1 |
| 32 | | | 2 | 1 | 2 | 1 | 1 |
| 33 | | | 2 | 2 | 2 | 2 | 2 |
| 34 | 2 | | | 2 | 2 | | |
| 35 | 2 | 1 | 1 | 2 | | 1 | |
| 36 | 1 | | 1 | 2 | 2 | | |
| 37 | 1 | | 1 | 2 | 2 | | |
| 38 | | 1 | | 2 | 2 | 2 | 2 |
| 39 | 2 | 2 | 2 | 2 | | | |
| 40 | | | | 2 | | 2 | 2 |
| 41 | | | | 2 | | 2 | 2 |
| 42 | | | | 2 | | 2 | 2 |
| 43 | | | | | | | |
| 44 | | | | | | | |
| 45 | | | | | | | |

**Claims**

1. An imidazole-5-carboximidate of general formula

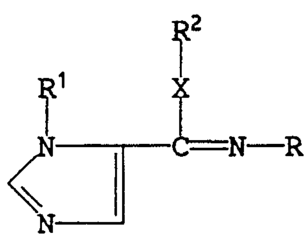

(I)

wherein R represents a phenyl group optionally substituted by a phenoxy group, a trifluoromethyl group, a methoxy group, a nitro group and/or by 1 to 5 halogen atoms; $R^1$ represents a $C_{1-6}$ alkyl group; $R^2$ represents a $C_{1-6}$ alkyl or haloalkyl group, a $C_{3-8}$ cycloalkyl group, a ($C_{3-8}$ cycloalkyl)methyl group, a $C_{3-6}$ alkenyl group, a phenyl group or a benzyl group; and X represents an oxygen or sulphur atom or a group $-NR^3-$, where $R^3$ represents a hydrogen atom or a methyl group or $R^2$ and $R^3$ together with the interjacent nitrogen atom represent a triazole or pyrrolidine ring.

**2.** A carboximidate according to Claim 1 wherein R is a phenyl group substituted by 1 to 3 halogen atoms or by a trifluoromethyl group and one or two halogen atoms.

**3.** A carboximidate according to Claim 1 or Claim 2 wherein said halogen atoms are chlorine or fluorine atoms.

**4.** A carboximidate according to any one of Claims 1, 2 and 3 wherein R is 2,4-dichlorophenyl, 2,4-difluorophenyl or 4-chloro-2-trifluoromethylphenyl, $R^1$ is a $C_{1-4}$ alkyl group, X is -O-, -S- or $-NCH_3-$, and $R^2$ is a $C_{1-4}$ alkyl or $C_{3-4}$ alkenyl group.

**5.** A process for the preparation of a carboximidate of formula I as defined in any one of Claims 1 to 4 which comprises reacting a compound of formula

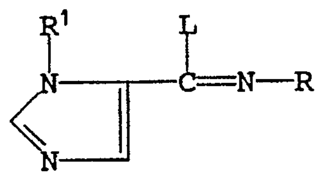

(II)

wherein R and $R^1$ are as defined in Claim 1 and L is a leaving group with a compound of formula

$$H-X-R^2 \qquad (III)$$

where $R^2$ and X are as defined in Claim 1, in the presence of a base.

**6.** A process according to Claim 5 wherein L is Cl or Br.

**7.** A fungicidal composition which comprises a carrier and, as active ingredient, a carboximidate according to any one of Claims 1 to 4.

**8.** A composition according to Claim 7, which comprises at least two carriers, at least one of which is a surface-active agent.

**9.** A method of combating fungus at a locus, which comprises treating the locus with a carboximidate of the general formula I as defined in any one of Claims 1 to 4.

**10.** A method as claimed in Claim 9, in which the locus comprises plants subject or subjected to fungal attack, seeds of such plants or the medium in which the plants are growing or are to be grown.

**11.** The use as a fungicide of a carboximidate of the general formula I as defined in any one of Claims 1 to 4.

## Revendications

**1.** Imidazole-5-carboxymidate de formule générale

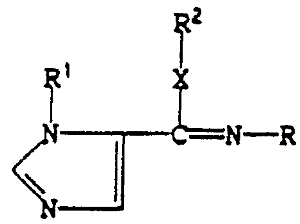

(I)

où R représente un groupe phényle éventuellement substitué par un groupe phénoxy, un groupe trifluorométhyle, un groupe méthoxy, un groupe nitro et/ou par de 1 à 5 atomes d'halogène; $R^1$ représente un groupe alcoyle en $C_{1-6}$; $R^2$ représente un groupe alcoyle en $C_{1-6}$ ou haloalcoyle, un groupe cycloalcoyle en $C_{3-8}$, un groupe (cycloalcoyle en $C_{3-8}$) méthyle, un groupe alcényle en $C_{3-6}$, un groupe phényle ou un groupe benzyle; et X représente un atome d'oxygène ou de soufre ou un groupe $-NR^3-$, où $R^3$ représente un atome d'hydrogène ou un groupe méthyle ou $R^2$ et $R^3$ ensemble avec l'atome d'azote situé entre eux représente un noyau triazole ou pyrrolidine.

2. Carboxymidate selon la revendication 1, où R est un groupe phényle substitué par de 1 à 3 atomes d'halogène ou par un groupe trifluorométhyle et un ou deux atomes d'halogène.

3. Carboxymidate selon la revendication 1 ou la revendication 2, où lesdits atomes d'halogène sont des atomes de chlore ou de fluor.

4. Carboxymidate selon l'une quelconque des revendications 1, 2, et 3, où R est un 2,4-dichlorophényle, 2,4-difluorophényle ou 4-chloro-2-trifluorométhylphényle, $R^1$ est un groupe alcoyle en $C_{1-4}$, X représente -O-, -S- ou $-NCH_3-$, et $R^2$ est un groupe alcoyle en $C_{1-4}$ ou alcényle en $C_{3-4}$.

5. Procédé de préparation d'un carboxymidate de formule I tel que défini dans l'une quelconque des revendications 1 à 4 dans lequel on fait réagir un composé de formule

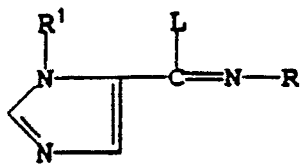

(II)

où R et $R^1$ sont tels que définis dans la revendication 1 et L est un groupe sortant, avec un composé de formule

$$H\text{-}X\text{-}R^2 \qquad (III)$$

où $R^2$ et X sont tels que définis dans la revendication 1, en présence d'une base.

6. Procédé selon la revendication 5, où L représente Cl ou Br.

7. Composition fongicide qui comprend un support et, comme ingrédient actif, un carboxymidate selon l'une des revendications 1 à 4.

8. Composition selon la revendication 7, qui comprend au moins deux supports, dont au moins un est un agent tensio-actif.

9. Procédé pour combattre les champignons en un lieu qui comprend le traitement de ce lieu avec un carbboxymidate de formule générale I tel que défini dans l'une quelconque des revendications 1 à 4.

**10.** Procédé selon la revendication 9, dans lequel le lieu comprend des plantes sujettes ou soumises à une attaque par les champignons, les semences de telles plantes ou le milieu dans lequel les plantes croissent ou sont mises à croître.

**11.** Application comme fongicide d'un carboxymidate de formule générale I tel que défini dans l'une quelconque des revendications 1 à 4.

### Ansprüche

**1.** Ein Imidazol-5-carboximidat der allgemeinen Formel

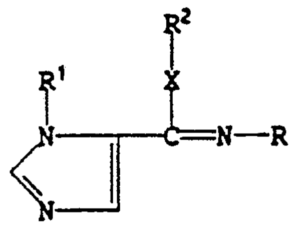

(I) ,

worin R eine Phenylgruppe, die gegebenenfalls durch eine Phenoxygruppe, eine Trifluormethylgruppe, eine Methoxygruppe, eine Nitrogruppe und/oder durch 1 bis 5 Halogenatome substituiert ist, darstellt; $R^1$ eine $C_{1-6}$-Alkylgruppe bedeutet; $R^2$ eine $C_{1-6}$-Alkyl- oder Halcgenalkylgruppe, eine $C_{3-8}$-Cycloalkylgruppe, eine ($C_{3-8}$-Cycloalkyl)methylgruppe, eine $C_{3-6}$-Alkenylgruppe, eine Phenylgruppe oder eine Benzylgruppe bedeutet; und X ein Sauerstoff- oder Schwefelatom oder eine Gruppe -$NR^3$- darstellt, worin $R^3$ ein Wasserstoffatom oder eine Methylgruppe bedeutet, oder $R^2$ und $R^3$ zusammen mit dem dazwischenliegended Stickstoffatom einen Triazol- oder Pyrrolidinring darstellen.

**2.** Carboximidat nach Anspruch 1, worin R eine durch 1 bis 3 Halogenatome oder durch eine Trifluormethylgruppe und 1 oder 2 Halogenatome substituierte Phenylgruppe bedeutet.

**3.** Carboximidat nach Anspruch 1 oder 2, worin die Halogenatome Chlor- oder Fluoratome sind.

**4.** Carboximidat nach eine der Ansprüche 1, 2 und 3, worin R für 2,4-Dichlorphenyl, 2,4-Difluorphenyl oder 4-Chlor-2-trifluormethylphenyl steht, $R^1$ eine $C_{1-4}$-Allylgruppe bedeutet, X für -O-, -S-oder -$NCH_3$- steht, und $R^2$ eine $C_{1-4}$-Alkylgruppe oder $C_{3-4}$-Alkenylgruppe darstellt.

**5.** Verfahren zur Herstellung eines Carboximidats der Formel I, wie in einem der Ansprüche 1 bis 4 definiert, welches ein Umsetzen einer Verbindung der Formel

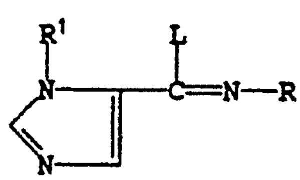

(II) ,

worin R und $R^1$ wie in Anspruch 1 definiert sind und L eine Leaving-Gruppe bedeutet, mit einer Verbindung der Formel

$$H\text{-}X\text{-}R^2 \qquad \text{(III)},$$

worin $R^2$ und X wie in Anspruch 1 definiert sind, in Anwesenheit einer Base umfaßt.

6. Verfahren nach Anspruch 5, worin L für Chlor oder Brom steht.

7. Fungizide Zusammensetzung, die einen Träger und als wirksamen Bestandteil ein Carboximidat nach einem der Ansprüche 1 bis 4 umfaßt.

8. Zusammensetzung nach Anspruch 7, die wenigstens zwei Träger umfaßt, von denen wenigstens einer ein oberflächenaktives Mittel ist.

9. Verfahren zur Bekämpfung von Pilzen an einem Ort, welches ein Behandeln des Ortes mit einem Carboximidat der allgemeinen Formel I, wie in einem der Ansprüche 1 bis 4 definiert, umfaßt.

10. Verfahren nach Anspruch 9, worin der Ort Pflanzen, die einem Pilzbefall unterliegen oder in Zukunft unterliegen, Sämlinge und Samen solcher Pflanzen oder das Medium umfaßt, worin die Pflanzen wachsen oder gezogen werden sollen.

11. Verwendung eines Carboximidats der allgemeinen Formel I, wie in einem der Ansprüche 1 bis 4 definiert, als Fungizid.